# EUROPEAN PATENT SPECIFICATION

(11) **EP 2 979 725 B1**
(45) Date of publication and mention of the grant of the patent: **19.09.2018**
(21) Application number: 14774167.2
(22) Date of filing: 25.03.2014
(51) Int. Cl.: A61M 25/00, A61L 29/06

(54) **TUBE FOR MEDICAL USE**
TUBUS ZUR MEDIZINISCHEN VERWENDUNG
TUBE À USAGE MÉDICAL

(30) Priority: 27.03.2013 JP 2013067328
(43) Date of publication of application: 03.02.2016
(73) Proprietor: Piolax Medical Devices, Inc., Yokohama-shi, Kanagawa 240-0023 (JP)
(72) Inventor: SAKAI, Shinichi, Yokohama-shi Kanagawa 240-0023 (JP); KOYAMA, Kazumi, Yokohama-shi Kanagawa 240-0023 (JP)
(74) Representative: Grünecker Patent- und Rechtsanwälte PartG mbB
(86) International application number: PCT/JP2014/058221
(87) International publication number: WO 2014/157164

(56) References cited:
- EP-A1- 1 712 247
- EP-A1- 1 721 631
- EP-A2- 0 956 878
- JP-A- 2000 279 508
- JP-A- 2006 218 070

## Description

### Technical Field

The present invention relates to a medical tube which is preferably indwelled in the body to administer a medical solution such as an anticancer agent.

### Background Art

To inject a medical solution such as an anticancer agent into the body, for example, a medical tube such as a catheter is percutaneously inserted into a tubular organ such as a blood vessel. Then, its tip end portion is moved to reach a portion affected by cancer so that the medical solution is administered from a discharge hole disposed in the tip end portion, or its tip end is moved to reach a central vein so that the medical solution is administered to the whole body is performed. Further, a medical solution injection port is connected to a base end portion of the medical tube, the medical tube is indwelled together with the medical solution injection port in the body, and a medical solution is periodically administered through the medical solution injection port.

As such a medical tube, Patent Literature 1 discloses a catheter which has an elongated tubular body having an outer diameter of 1.35 mm to 3 mm. The catheter has a tip end portion and a base end portion. In addition, the catheter has an inner layer forming the inner surface of the tubular body, an outer layer forming the outer surface of the tubular body, and plural reinforcing wires embedded between the inner surface and the outer surface. In the catheter, the reinforcing wires have a width that is substantially parallel to the circumferential direction of the tubular body, and a thickness that is substantially parallel to a radial direction of the tubular body. A ratio of the wall thickness of the tubular body to the thickness of the reinforcing wires is 3.5 to 3.8, and a proportion of the total cross-sectional area of the plural reinforcing wires to the cross-sectional area of the tubular body is not small than 17% and smaller than 25%.

As the inner layer, a fluorine resin such as polytetrafluoroethylene is preferred. As the outer layer, various thermoplastic elastomers based on styrene, polyolefin, polyurethane, polyester, polyamide, polybutadiene, transpolyisoprene, fluorine rubber, chlorinated polyethylene, and the like are preferred. As the reinforcing wires, a metal such as stainless steel is preferred.

### Citation List

### Patent Literature

**Patent Literature 1:** JP-2006-288670-A

### Summary of Invention

### Technical Problem

As a medical tube to be indwelled in the body in order to inject a medical solution such as an anticancer agent into, flexibility is requested so as to fit with bends of a tubular organ such as a blood vessel, and to flexibly follow the movement. As such medical tube kink resistance is also requested so as to be prevented from being breaking even when bent, prevents from kinking and clogging, and breakage resistance and bending durability which, when the patient moves and a strong force is applied to the tube, or when the tube is repeatedly bent, prevents from breaking. To embed a medical tube in the body while being connected to a medical solution injection port, the tube is cut to a length which is adequate to the embedding-target site in the patient, and the medical solution injection port is connected to the cut end surface.

In a medical tube as disclosed in Patent Literature 1, reinforcing wires made of a metal such as stainless steel are embedded between the inner layer and the outer layer. Therefore, the medical tube is excellent in kink resistance, pressure resistance, torque transmission ability, pushing ability, and the like. In the case where such a medical tube is to be used as an indwelling-purpose medical tube and to be indwelled in a largely/complicatedly-bent tubular organ, however, the kink resistance of the medical tube may still be insufficient. In the case where such a medical tube is embedded to be connected to a medical solution injection port, when the tube is cut to a length which is adequate to the embedding-target site in the patient, the opening in the cut surface is collapsed to a flat condition, and end portions of metal reinforcing wires project from the cut surface, thereby producing a disadvantage that, when no countermeasure is taken, the medical tube cannot be connected to the medical solution injection port.

An object of the invention is to provide a medical tube in which kink resistance and breakage resistance can be enhanced while maintaining flexibility, and the opening in the cut surface is prevented from collapsing even when the tube is cut, and end portions of reinforcing wires are prevented from projecting from the cut surface.

EP 0 956 878 describes a tubular catheter main segment comprising an inner layer, a reinforcement layer formed from reinforcement yarns disposed on the inner layer, and an outer layer. The inner and outer layers are adhered to each other, with the reinforcement yarns there between, and partially embedded in both layers. The reinforcement yarns are preferably from polyamide, whereas the inner and outer layers are preferably from polyurethane.

### Solution to Problem

To attain the object, the invention provides
a medical tube including:an inner layer tube which is made of polyurethane;a reinforcing member which is configured by wire members formed from a material containing polyethylene terephthalate and/or polyamide, and which is attached to an outer circumference of the inner layer tube; and
an outer layer tube which is made of polyurethane, and which covers the inner layer tube from an outside thereof so as to embed the reinforcing member,wherein, in a cut surface which is perpendicular to an axial direction of the medical tube, a ratio of a cross-sectional area of the reinforcing member to cross-sectional areas of the inner layer tube and the outer layer tube is 5 to 43%, and wherein a thickness of the outer layer tube is 2 to 6 times a thickness of the inner layer tube.

The invention may provide the medical tube,
wherein the medical tube is used while being connected to a medical solution
injection port.

### Advantageous Effects of Invention

According to the medical tube of the invention, the reinforcing member configured by wire members formed from a material containing polyethylene terephthalate and/or polyamide is embedded between the inner and outer layer tubes made of polyurethane, and in a cut surface which is perpendicular to the axial direction of the medical tube, the ratio of the cross-sectional area of the reinforcing member to the cross-sectional areas of the inner layer tube and the outer layer tube is 5 to 43%. Therefore, kink resistance and breakage resistance can be enhanced without lowering flexibility.

Even in the case where the medical tube is indwelled in a largely/complicatedly-bent tubular organ, the excellent kink resistance can suppress clogging, breaking, and the like of the tube, and even when the tube is indwelled in a bent condition for a long time period, the excellent flexibility can suppress bending fatigue of the tube, disadvantages due to the fatigue, and the like.

### Brief Description of Drawings

Fig. 1 is a perspective view showing an embodiment of the medical tube of the invention.
Figs. 2A and 2B show the medical tube. Fig. 2A is a sectional view of a cut surface which is perpendicular to the axial direction. Fig. 2B is a sectional view of a cut surface parallel to the axial direction.
Figs. 3A to 3C show other examples of the medical tube. Fig. 3A is a sectional view showing a first modification. Fig. 3B is a sectional view showing a second modification. Fig. 3C is a sectional view showing a third modification.
Fig. 4 is a perspective view showing an embodiment of the indwelling-purpose catheter device of the invention.
Fig. 5 is a diagram showing a method of using the medical tube of the invention, and the indwelling-purpose catheter device of the invention in which the medical tube is used.
Fig. 6 is a view illustrating a test of kink resistance of a medical tube.
Fig. 7 is a view illustrating a test of bending durability of a medical tube.

### Description of Embodiments

Hereinafter, an embodiment of the medical tube of the invention will be described with reference to the drawings.

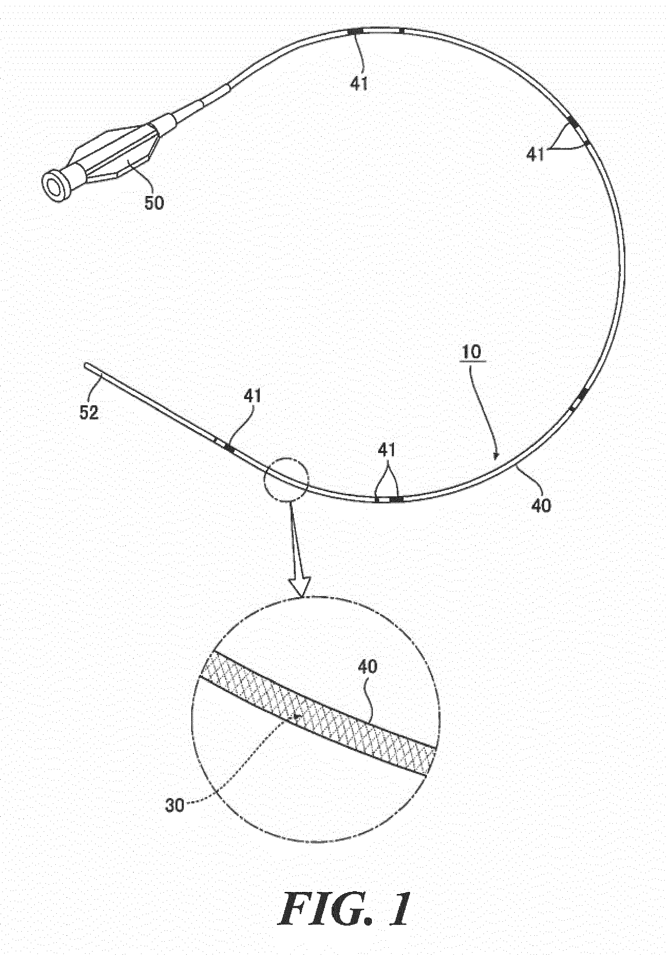

As shown in Figs. 1 to 2B, the medical tube 10 of the embodiment includes: an inner layer tube 20; a reinforcing member 30 which is attached to the outer circumference of the inner layer tube 20; and an outer layer tube 40 which covers the outside of the inner layer tube 20 and embeds the reinforcing member 30. In the medical tube, a hub 50 for inserting a guide wire or the like is connected to a base end portion.

The inner layer tube 20 is formed from polyurethane such as polyether type polyurethane, polyester type polyurethane, polycarbonate type polyurethane, or polycaprolactone type polyurethane.

Preferably, the hardness, which is specified in JIS K 6253 and measured by a durometer, of the inner layer tube 20 is 60A to 90D, and more preferably 74A to 75D.

The inner layer tube 20 may contain bismuth tungstate, powdered tungsten, powdered tantalum, barium sulfate, bismuth trioxide, bismuth subcarbonate, basic bismuth carbonate, or a combination of these, to be X-ray opaque. A wire member made of an X-ray opaque metal such as platinum, a platinum-iridium alloy, a platinum-tungsten alloy, a platinum-nickel alloy, gold, or silver may be wound around the inner layer tube 20; a tube made of the X-ray opaque metal may be attached to the outer circum ference of the inner layer tube 20; or a plate made of the X-ray opaque metal may be formed into an annular shape, and then attached to the outer circumference of the inner layer tube 20, so that these members function as X-ray opaque markers. The X-ray opaque markers may be attached to the outer circumference of the reinforcing member 30 which will be described later, or that of the outer layer tube 40.

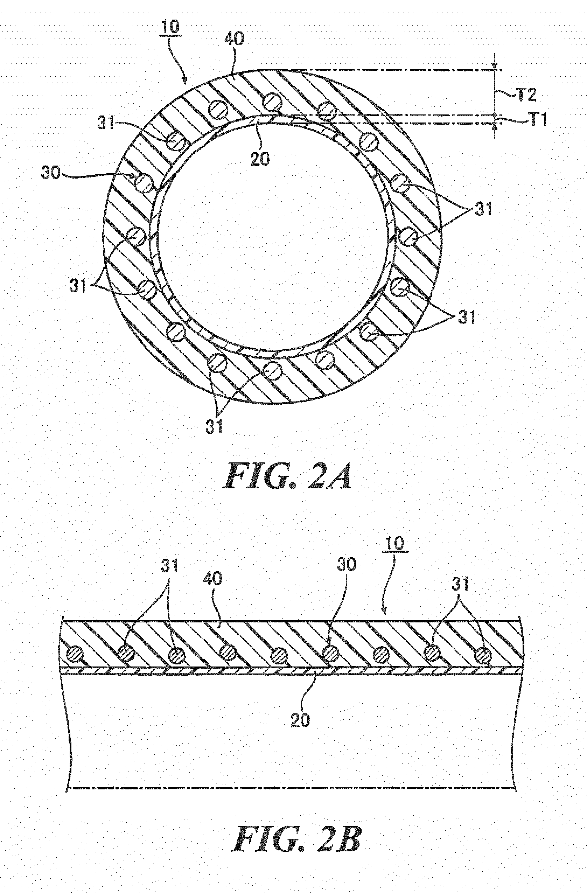

As shown in Fig. 2A, the inner layer tube 20 preferably has an inner diameter of 0.27 to 4.08 mm, more preferably, 0.45 to 1.90 mm, preferably has an outer diameter of 0.28 to 4.29 mm, more preferably, 0.47 to 2.00 mm, and preferably has a thickness T1 (see Fig. 2A) of 0.01 to 0.21 mm, more preferably, 0.02 to 0.10 mm.

The inner circumference of the inner layer tube 20 may be coated with a physiologically active substance having a fibrinolytic activity, such as urokinase, or a hydrophilic resin such as 2-methacryloyloxyethyl phosphorylcholine (MPC) polymer, polyvinylpyrrolidone, polyethyleneglycol, or methylvinylether-maleic anhydride copolymer, and further with an antimicrobial agent, pigment/dye (colorant), and the like. A part or the whole of the inner layer tube 20 may be colored or printed with marks such as depth marks, and the reinforcing member 30 and the outer layer tube 40 which are on the outer circumference of the inner layer tube may be colored in a transparent color or a visible color, thereby making the inner layer tube visible from the outside of the medical tube 10 is formed.

On the other hand, the reinforcing member 30 is configured by wire members 31 (see Figs. 2A and 2B) formed from a material containing polyethylene terephthalate and/or polyamide. For example, the polyamide may be nylon 6, nylon 9, nylon 11, nylon 12, nylon 46, nylon 64, nylon 66, nylon 610, nylon 612, a material which is obtained by softening such nylon with plasticizer, or a mixture of these. Alternatively, the reinforcing member may be formed by combining two or more kinds of wire members 31 which are made of different materials.

In the reinforcing member 30 in the embodiment, on the outer circumference of the inner layer tube 20, the wire members 31 are spirally knitted at predetermined pitches to be formed into a tubular shape having plural meshes such that the wire members 31 are arranged substantially uniformly in the circumferential direction of the inner layer tube 20 (see Fig. 2A), and attached to the outer circumference of the inner layer tube 20.

Alternatively, the reinforcing member 30 may be configured by previously knitting the wire members 31 into a tubular shape, and attaching the wire members to the outer circumference of the inner layer tube 20 by an adhesive agent or the like, or by attaching plural wire members 31 in a predetermined arrangement pattern onto the outer circumference of the inner layer tube 20 by an adhesive agent or the like. The configuration is not particularly limited.

The "knitting" of the wire members 31 includes weaving, braiding, tangling, simply crossing and the like. Intersecting portions of the wire members 31 may be joined together, or may not be joined so as to be freely moved.

As shown in Figs. 2A and 2B, the wire members 31 in the embodiment have a circular sectional shape. Alternatively, the wire members may have a rectangular sectional shape. Preferably, the diameter of the wire members 31 is 0.01 to 0.20 mm, and more preferably 0.03 to 0.15 mm. In the case where the reinforcing member 30 is formed into a tubular shape, the outer diameter is preferably 0.29 to 4.69 mm, and more preferably 0.50 to 2.30 mm.

The wire members 31 may contain bismuth tungstate, powdered tungsten, powdered tantalum, barium sulfate, bismuth trioxide, bismuth subcarbonate, basic bismuth carbonate, or a combination of these, to be X-ray opaque.

A part or the whole of the reinforcing member 30 may be colored or printed with marks such as depth marks, and the outer layer tube 40 may be colored in a transparent color or a color which allows the reinforcing member 30 to be visible, thereby making the reinforcing member 30 and patterns etc. formed thereon visible from the outside of the medical tube 10.

The outer layer tube 40 is formed from polyurethane such as polyether type polyurethane, polyester type polyurethane, polycarbonate type polyurethane, or polycaprolactone type polyurethane. The outer layer tube 40 enters meshes of the reinforcing member 30 at the outer circumference of the inner layer tube 20, and the outer layer tube 40 covers the outside of the inner layer tube 20 so as to embed the reinforcing member 30.

Preferably, the hardness, which is which is specified under JIS K 6253 and measured by a durometer, of the outer layer tube 40 is 60A to 90D, and more preferably 74A to 75D. Preferably, the outer layer tube 40 is softer than the inner layer tube 20.

In the embodiment, as shown in Fig. 1, plural marks 41 are printed on the outer circumference of the outer layer tube 40 along the axial direction at predetermined intervals, to constitute depth marks which are used as indexes of the insertion depth of the medical tube 10. As described above, alternatively, such marks 41 are formed on the reinforcing member 30, and viewed through the outer layer tube 40. A part or the whole of the outer layer tube 40 may be colored.

The outer layer tube 40 may contain bismuth tungstate, powdered tungsten, powdered tantalum, barium sulfate, bismuth trioxide, bismuth subcarbonate, basic bismuth carbonate, or a combination of these, to be X-ray opaque. Alternatively, X-ray opaque markers configured by wire members, tubes, annularly formed plates, or the like which are formed from the above-described X-ray opaque metal may be attached to the outer circumference of the outer layer tube 40.

The outer layer tube 40 preferably has an inner diameter of 0.28 to 4.29 mm, more preferably 0.47 to 2.00 mm, and preferably has an outer diameter of 0.41 to 6.18 mm, more preferably 0.69 to 2.88 mm. The outer layer tube 40 preferably has a thickness T2 (see Fig. 2A) of 0.06 to 0.84 mm, more preferably 0.09 to 0.39 mm.

According to the invention, the thickness T2 of the outer layer tube 40 is 2 to 6 times the thickness T1 of the inner layer tube 20, and more preferably 3 to 5 times. When the thickness T2 is less than 2 times the thickness T1, the thickness T2 of the outer layer tube 40 may not be sufficient to reliably embed the reinforcing member 30 (the reinforcing member 30 may project from the outer layer tube 40). When the thickness T2 is larger than 6 times the thickness T1, the whole outer diameter of the medical tube 10 is excessively large.

The outer circumference of the outer layer tube 40 may be coated with a physiologically active substance having a fibrinolytic activity, such as urokinase, or a hydrophilic resin such as 2-methacryloyloxyethyl phosphorylcholine (MPC) polymer, polyvinylpyrrolidone, polyethyleneglycol, or methylvinylether-maleic anhydride copolymer, and further with an antimicrobial agent, pigment/dye (colorant), and the like.

In the medical tube 10, in the cut surface which is perpendicular to the axial direction, a ratio of the cross-sectional area of the reinforcing member 30 to the cross-sectional areas of the inner layer tube 20 and the outer layer tube 40 (a ratio in the case where the cross-sectional areas of the inner layer tube 20 and the outer layer tube 40 are set to 100%) is 5 to 43% (see Fig. 2A). When the ratio is less than 5%, kink resistance cannot be sufficiently obtained. When the ratio exceeds 43%, the joining area between the reinforcing member 30, and the inner layer tube 20 and the outer layer tube 40 is reduced, the reinforcing member 30 is easily peeled off, and durability against bending is low.

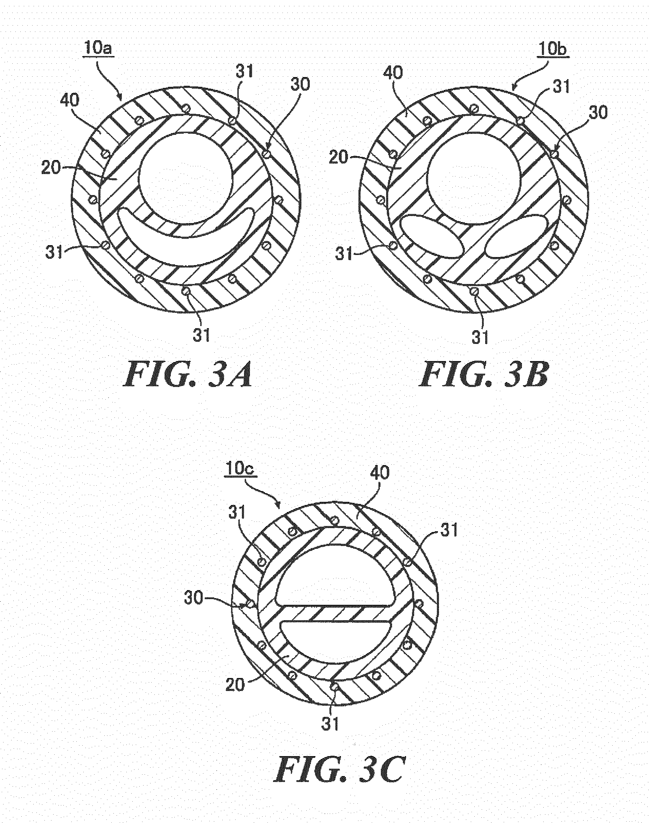

In the above-described medical tube 10, as shown in Fig. 2A, the inner layer tube 20 has only one lumen which is circular in section. Alternatively, the inner layer tube may have plural lumens as shown in Figs. 3A to 3B. In a medical tube 10a shown in Fig. 3A, for example, the inner layer tube 20 has two lumens, i.e., a circular lumen and a lumen having a substantially crescentic shape. The inner layer tube 20 of a medical tube 10b shown in Fig. 3B has three lumens, i.e., a circular lumen and a pair of lumens having a substantially oval shape. The inner layer tube 20 of a medical tube 10c shown in Fig. 3C has a pair of substantially-semicircular-shaped lumens. The lumens have different sizes, and are opposed to each other.

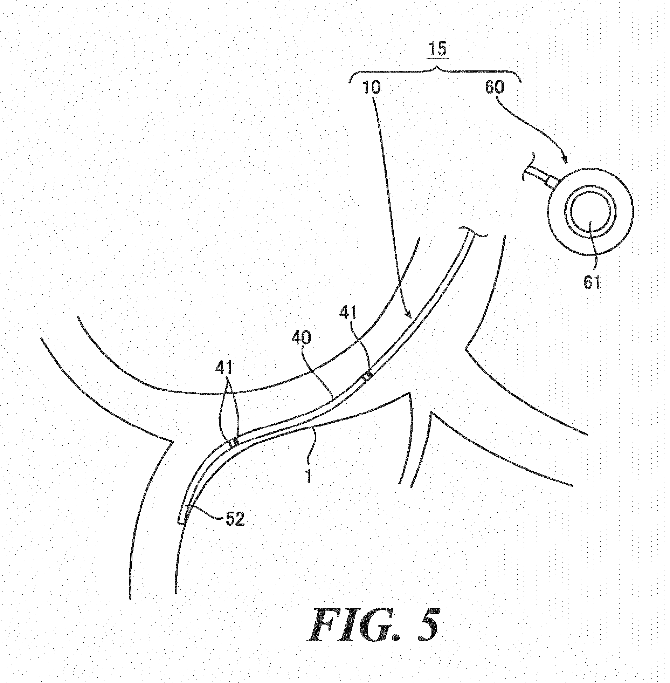

The medical tube 10 of the embodiment is used as an indwelling-purpose catheter which is to be indwelled in a tubular organ such as a blood vessel to administer a medical solution or the like (see Fig. 5). For example, the medical tube may be used as a shunt tube for draining a fluid accumulated in the ventricle into the abdominal cavity, a drainage tube to be inserted into the bile duct to drain the bile or the like, a catheter for carrying a stent, a dialysis catheter, and the like.

For example, this medical tube 10 can be produced in the following manner.

First, the outer circumference of a core rod configured by a metal rod or the like is covered with an inner-layer-tube resin material by extrusion molding to form the inner layer tube 20. Alternatively, the inner layer tube 20 is formed by extrusion molding the inner-layer-tube resin material, and the core rod is inserted into the inner circumference thereof. Next, the wire members 31 are knitted on the outer circumference of the inner layer tube 20 to form the tubular reinforcing member 30, or the reinforcing member 30 configured by the wire members 31 which are previously formed into a tubular shape is attached to the outer circumference of the inner layer tube 20. Furthermore, the reinforcing member 30 is covered with an outer-layer-tube resin material by extrusion molding or the like from the outer circumference thereof. As a result, the outer layer tube 40 is formed so as to enter the meshes of the reinforcing member 30 and so as to embed the reinforcing member 30. In the case where the tip end wall having the incision is disposed in the tip end portion 52, the tip end portion 52 which is separately formed is joined to the tip end of the tube which is obtained in the above-described steps, thereby producing the medical tube 10.

The method of producing the medical tube 10 is not limited to the above-described methods. For example, the outer layer tube 40 may be configured by a heat shrinkable tube, and heat-shrunk on the outer circumference of the reinforcing member 30 to thereby cover and embed the reinforcing member 30.

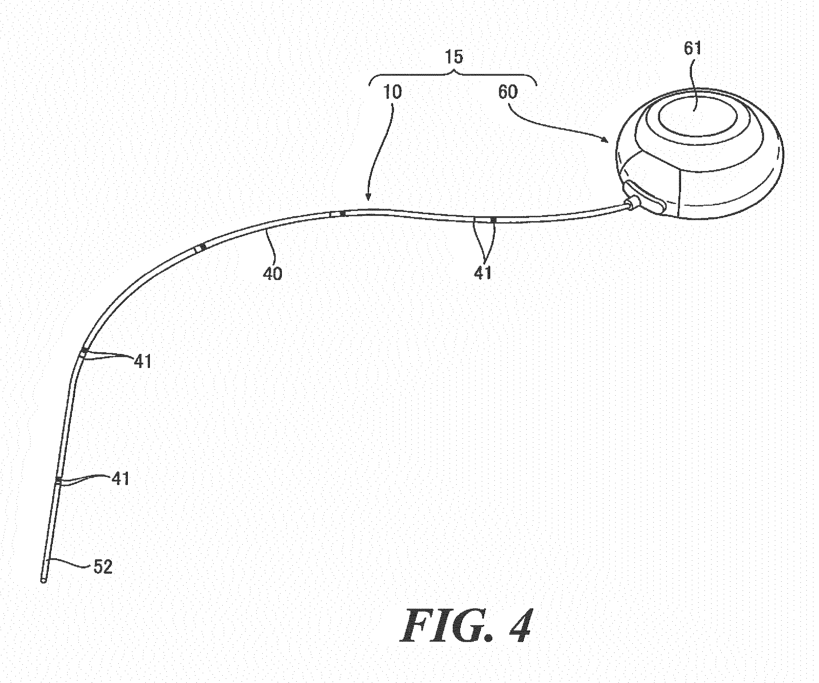

As shown in Fig. 4, a medical solution injection port 60 may be connected to a base end portion of the medical tube 10, and the medical tube may be used as an indwelling-purpose catheter device 15. The indwelling-purpose catheter device 15 is indwelled continuously or temporarily in a tubular organ 1 of the human body such as a blood vessel (see Fig. 5), and used for, as required, administering an anticancer agent, a nutrient, or the like.

Furthermore, the medical tube 10 of the invention is configured so that, after its tip end portion is inserted into a predetermined site of a tubular organ in the body, the base end side of the medical tube 10 is cut to be adjusted to the optimum length which allows the tube to pass through a comfortable route, and then to be connected to the medical solution injection port 60, and the medical solution injection port 60 is connected to the cut end portion. The medical solution injection port 60 has a rubber film 61 through which the needle of a syringe can be pierced, and when a syringe needle which is not shown is inserted from the outside of the body, a medical solution or the like can be supplied to the medical solution injection port 60 through the rubber film 61.

Next, an example of the method of using this medical tube 10 and an indwelling-purpose catheter device which employs the medical tube 10 will be described.

Firstly, a puncture needle which is not shown is thrust into a predetermined tubular organ by the well-known Seldinger technique, and a guide wire which is not shown is introduced from the base end of the puncture needle to be inserted into the tubular organ 1. Thereafter, the puncture needle is pulled out, and a scabbard-like sheath which is not shown is inserted into the tubular organ 1 along the guide wire. Next, the base end of the guide wire is inserted through the opening of the tip end portion 52 of the medical tube 10, and the guide wire is introduced into the medical tube 10.

Then, the tip end of the guide wire is advanced to a target site in the tubular organ 1, the medical tube 10 is inserted into the sheath along the outer circumference of the guide wire, and a tip end portion of the tube is caused to reach the target site in the tubular organ 1. Thereafter, the guide wire is pulled out from the medical tube 10, the sheath is pulled out from the tubular organ 1, a base portion side of the medical tube 10 to which the hub 50 is connected is cut in accordance with the application site, the body shape of the patient, and the like, and the medical solution injection port 60 is connected to the cut base end portion. Thereafter, the skin is incised, and the medical solution injection port 60 is embedded under the skin (see Fig. 5).

In this way, the medical solution injection port 60 is embedded in the body of the patient, the needle of a syringe which is not shown is pierced through the rubber film 61 of the medical solution injection port 60, and a fluid such as an anticancer agent is injected, whereby the fluid such as an anticancer agent can be injected into the medical tube 10, and discharged from the opening of the tip end portion 52 of the medical tube 10 to be administered to the target site of the tubular organ 1.

In the medical tube 10, the reinforcing member 30 which is configured by the wire members 31 formed from a material containing polyethylene terephthalate and/or polyamide, and is embedded between the inner layer tube 20 and the outer layer tube 40 which are made of polyurethane. In a cut surface which is perpendicular to the axial direction, a ratio of a cross-sectional area of the reinforcing member 30 to cross-sectional areas of the inner layer tube 20 and the outer layer tube 40 is 5 to 43% as shown in Fig. 2A. Therefore, the reinforcing member 30 can be firmly joined to the inner layer tube 20 and the outer layer tube 40, and sufficient kink resistance and breakage resistance can be obtained without lowering flexibility.

Even in the case where, as shown in Fig. 5, the medical tube 10 is indwelled in the largely/complicatedly-bent tubular organ 1, the excellent kink resistance and breakage resistance can suppress clogging, breaking, and the like of the lumen of the medical tube 10, and even when the tube is indwelled in a bent condition for a long time period, the excellent flexibility can suppress bending fatigue and the like of the medical tube 10, and disadvantages such as breakage of the medical tube 10.

The thickness T2 of the outer layer tube 40 is 2 to 6 times the thickness T1 of the inner layer tube 20 (see Fig. 2A). While the outer layer tube 40 ensures the thickness T2 which is necessary for embedding the reinforcing member 30, therefore, the thickness T1 of the inner layer tube 20 can be reduced as far as possible. While maintaining the bonding property between the inner layer tube 20 and the outer layer tube 40, and the reinforcing member 30, consequently, the wall thickness of the medical tube 10 can be reduced as far as possible, and while ensuring the inner diameter of the medical tube 10, the outer diameter of the medical tube 10 can be made relatively small.

On the other hand, in the indwelling-purpose catheter device 15 which uses the medical tube 10, the reinforcing member 30 which is placed between the inner layer tube 20 and the outer layer tube 40 is configured by the wire members 31 formed from a material containing polyethylene terephthalate and/or polyamide. When, during surgery, the medical tube 10 is cut so that the length is adjusted in accordance with the application site or the body shape of the patient, therefore, the reinforcing member 30 can be prevented from projecting from the end surface as in the case where metal wire members made of stainless steel or the like are cut, and the cut end surface can be prevented from collapsing. Consequently, the tube can be smoothly connected to the medical solution injection port 60, and the work of connecting the medical solution injection port 60 can be performed safely and promptly.

### Examples

Medical tubes having the structure shown in Figs. 2A and 2B are tested for kink resistance, durability (bending durability) when bending stress is repeatedly applied, and breakage resistance.

### (1) Preparation of samples

Examples 1 to 9 and Comparative examples I to 4 are produced under the conditions shown in Table I below.

### (2) Test for kink resistance

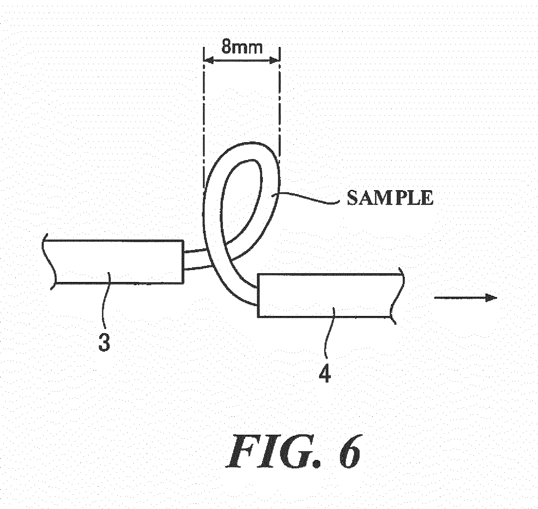

As shown in Fig. 6, one end portion of each sample is inserted into and fixed to a metal tube 3, the other end is fixed to another metal tube 4, and an intermediate portion extracted from the metal tubes 3, 4 is bent into a loop shape, and pulled at a constant rate. When the outer diameter of the loop became 8 mm, it is tested whether kink occurs or not. Table I shows the results. In Table 1, ZZ indicates a case where kink did not occur, and XX indicates a case where kink occurred.

### (3) Test for bending durability

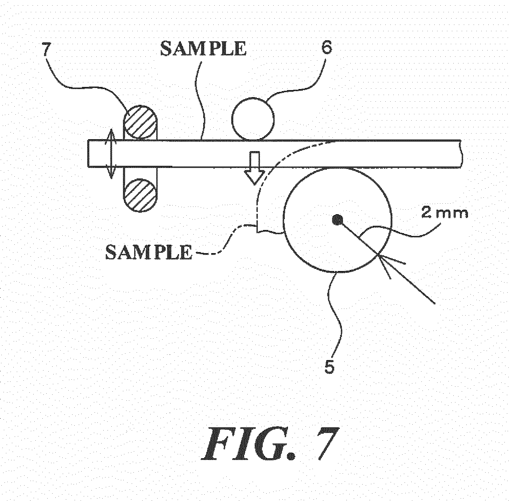

As shown in Fig. 7, each sample is placed on a support rod 5 having a radius of 2 mm, and fixed by a fixing tool which is not shown. An operation in which an upper portion of each sample is pressed down by a press roller 6 to be bent along the outer circumference of the support rod 5 (see the phantom line), thereafter the press roller 6 is lifted up to return the initial position, and after the sample elastically returns to a straight condition, the sample is again pressed down by the press roller 6 to be bent is repeated 720,000 times in a state where the sample is immersed in a bicarbonate Ringer solution at a temperature of 37°C.

A vertically-movable frame-like push-up member 7 is placed on the outer circumference of a tip end portion of each sample. After bending by the press roller 6, the member is lifted up to forcibly return the sample to the initial straight condition so as to provide a configuration where no time lag occurs between the bending of each sample, and the elastic returning of the sample.

After each sample is press bent 720,000 times by the press roller 6 as described above, the sample is detached from the support rod 5. The sample is checked by tactile and visual inspection whether there is a problem in durability or not. Table 1 shows the results. In Table 1, ZZ indicates a case where there is no problem in durability, and XX indicates a case where there is a problem.

### (4) Test for breakage resistance

Each sample is set to a tensile tester, pulled at a constant tension speed, and tested whether, when the tension reaches 15 N, the sample breaks or not. Table 1 shows the results. In Table 1, ZZ indicates a case where breakage did not occur, and XX indicates a case where breakage occurred. In Table 1, PA means polyamide, and PET means polyethylene terephthalate.

**[Table 1]**

| | | Example 1 | Example 2 | Example 3 | Example 4 | Comparative Example 1 | Comparative Example 2 | Example 5 | Example 6 | Example 7 | Example 8 | Example 9 | Comparative Example 3 | Comparative Example 4 |
|---|---|---|---|---|---|---|---|---|---|---|---|---|---|---|
| Dimension (whole tube) | Outer diameter (mm) | 2.06 | 2.06 | 2.04 | 1.65 | 2.06 | 2.06 | 167 | 2.10 | 2.06 | 2.02 | 2.06 | 1.67 | 2.06 |
| | Inner diameter (mm) | 1.33 | 1.35 | 1.33 | 1.02 | 1.36 | 1.36 | 1.08 | 1.32 | 1.36 | 1.40 | 1.36 | 1.08 | 1.36 |
| Material of reinforcing member | | PA | PA | PA | PA | PET | PET | PET | PET | PET | PET | PET | PET | PET |
| Ratio of cross-sectional area of reinforcing member to cross-sectional areas of inner and outer tubes | | 0.14 | 0.15 | 0.15 | 0.22 | 0.03 | 0.04 | 0.05 | 0.06 | 0.07 | 0.08 | 0.43 | 0.80 | 1.15 |
| Kink resistance | | ZZ | ZZ | ZZ | ZZ | XX | XX | ZZ | ZZ | ZZ | ZZ | ZZ | ZZ | ZZ |
| breakage resistance | | ZZ | ZZ | ZZ | ZZ | ZZ | ZZ | ZZ | ZZ | ZZ | ZZ | ZZ | ZZ | ZZ |
| Bending durability | | ZZ | ZZ | ZZ | ZZ | ZZ | ZZ | ZZ | ZZ | ZZ | ZZ | ZZ | XX | XX |

### (5) Test results

As shown in Table I above, Comparative examples 1 and 2 are insufficient in kink resistance, and Comparative examples 3 and 4 are problematic in bending durability. On the other hand, it is confirmed that Examples 1 to 9 have sufficient kink resistance and bending durability, and have no problem in breakage resistance.

### Reference Signs List

- 10: medical tube
- 15: indwelling-purpose catheter device
- 20: inner layer tube
- 30: reinforcing member
- 31: wire member
- 40: outer layer tube
- 60: medical solution injection port

## Claims

1. A medical tube (10) including:
an inner layer tube (20) which is made of polyurethane;
a reinforcing member (30) which is configured by wire members (31) formed from a material containing polyethylene terephthalate and/or polyamide, and which is attached to an outer circumference of the inner layer tube (20); and
an outer layer tube (40) which is made of polyurethane, and which covers the inner layer tube (20) from an outside thereof so as to embed the reinforcing member (30),
wherein, in a cut surface which is perpendicular to an axial direction of the medical tube (10), a ratio of a cross-sectional area of the reinforcing member (30) to cross-sectional areas of the inner layer tube (20) and the outer layer tube (40) is 5 to 43%, and
wherein a thickness of the outer layer tube (40) is 2 to 6 times a thickness of the inner layer tube (20).

2. The medical tube (10) of claim 1, wherein the outer layer tube (40) is softer than the inner layer tube (20).

3. The medical tube (10) of Claim 1 or 2, wherein the medical tube (10) is used while being connected to a medical solution injection port (60).

## Patentansprüche

1. Medizinischer Schlauch (10) beinhaltend:
einen Innenschichtschlauch (20), der aus Polyurethan hergestellt ist;
ein Verstärkungselement (30), das durch Drahtelemente (31) gestaltet ist, die aus einem Material gebildet sind, das Polyethylenterephthalat und/oder Polyamid enthält und das an einem äußeren Umfang des Innenschichtschlauchs (20) befestigt ist; und
einen Außenschichtschlauch (40), der aus Polyurethan hergestellt ist und der den Innenschichtschlauch (20) von einer Außenseite davon bedeckt, um das Verstärkungselement (30) einzubetten,
wobei in einer Schnittfläche, die senkrecht zu einer Axialrichtung des medizinischen Schlauchs (10) ist, ein Verhältnis einer Querschnittsfläche des Verstärkungselements (30) zu Querschnittsflächen des Innenschichtschlauchs (20) und des Außenschichtschlauchs (40) 5 bis 43% beträgt und
wobei die Dicke des Außenschichtschlauchs (40) 2 bis 6 mal die Dicke des Innenschichtschlauchs (20) beträgt.

2. Medizinischer Schlauch (10) nach Anspruch 1, wobei der Außenschichtschlauch (40) weicher als der Innenschichtschlauch (20) ist.

3. Medizinischer Schlauch (10) nach Anspruch 1 oder 2, wobei der medizinische Schlauch (10) verwendet wird während er mit einer Injektionsöffnung (60) für eine medizinische Lösung verbunden ist.

## Revendications

1. Tube médical (10) incluant :
un tube formant couche interne (20) qui est fait de polyuréthane ;
un membre de renforcement (30) qui est configuré par des membres en fil (31) formés en un matériau contenant du polyéthylène téréphtalate et/ou un polyamide, et qui est fixé à une circonférence externe du tube formant couche interne (20) ; et
un tube formant couche externe (40) qui est fait de polyuréthane, et qui couvre le tube formant couche interne (20) depuis un côté externe de celui-ci de manière à noyer le membre de renforcement (30),
où, dans une surface de coupe qui est perpendiculaire à une direction axiale du tube médical (10), un rapport d'une aire en section droite du membre de renforcement (30) aux aires en section droite du tube formant couche interne (20) et du tube formant couche externe (40) est 5 à 43 %, et
où une épaisseur du tube formant couche externe (40) est 2 à 6 fois une épaisseur du tube formant couche interne (20).

2. Tube médical (10) selon la revendication 1, où le tube formant couche externe (40) est plus souple que le tube formant couche interne (20).

3. Tube médical (10) selon la revendication 1 ou 2, où le tube médical (10) est utilisé tandis qu'il est relié à un orifice d'injection de solution médicale (60).
